(19) Europäisches Patentamt

European Patent Office

Office européen des brevets

(11) **EP 0 966 919 B1**

(12) **EUROPEAN PATENT SPECIFICATION**

(45) Date of publication and mention
of the grant of the patent:
**11.08.2004 Bulletin 2004/33**

(51) Int Cl.[7]: **A61B 8/00**, A61B 5/04,
A61B 5/00, A61B 5/0484
// G06F17/14

(21) Application number: **96945916.3**

(22) Date of filing: **26.12.1996**

(86) International application number:
**PCT/RU1996/000364**

(87) International publication number:
**WO 1998/019601 (14.05.1998 Gazette 1998/19)**

(54) **METHOD FOR INFLUENCING THE ORGANISM**

VERFAHREN ZUM BEEINFLUSSEN DES ORGANISMUS

PROCEDE PERMETTANT D'AGIR SUR L'ORGANISME

(84) Designated Contracting States:
**AT BE CH DE DK ES FI FR GB GR IE IT LI LU MC
NL PT SE**

(30) Priority: **01.11.1996 RU 96121164**

(43) Date of publication of application:
**29.12.1999 Bulletin 1999/52**

(73) Proprietor: **Levin, Yakov Iosifovich
Moscow, 129272 (RU)**

(72) Inventor: **Levin, Yakov Iosifovich
Moscow, 129272 (RU)**

(74) Representative: **BOVARD AG - Patentanwälte
Optingenstrasse 16
3000 Bern 25 (CH)**

(56) References cited:
EP-A- 0 301 790          WO-A-87/01023
DE-A- 2 908 187          DE-A- 3 328 599
DE-A- 3 331 458          SU-A- 1 725 828
SU-A- 1 745 204          SU-A- 1 780 716
US-A- 3 565 058          US-A- 3 910 258
US-A- 3 913 567          US-A- 3 924 606

• KNAPP R B ET AL: "A REAL-TIME DIGITAL
SIGNAL PROCESSING SYSTEM FOR
BIOELECTRIC CONTROL OF MUSIC"
INTERNATIONAL CONFERENCE ON
ACOUSTICS, SPEECH & SIGNAL PROCESSING.
ICASSP,US,NEW YORK, IEEE, vol. CONF. 13,
page 2556-2557 XP000012478

## Description

Technical Field

The invention relates to a method for registering a sound which is useful for influencing the human body for bioadaptive correction of man's functional condition and a respective registering device which is useful in the area of medicine.

Prior Art

Known from the level of technology are methods of influencing the body by biological feedback, where biopotentials, mainly of brain electrical activity are recorded, transformed and the obtained electroencephalogram (EEG) is processed to isolate, from the spectrum, a certain frequency band that corresponds to alpha-rhythm, and then a control signal is formed generating sound effect on the body with the level proportional to alpha-rhythm in the EEG spectrum (see USSR Authorship Certificate No. 1124922, class A 61 B 5\04, 1984; USSR Authorship Certificate No. 1780716, class A 61 B 5\04, 1992; US Patent No. 3896790, class A 61 B 5\04, 1975).

Psychophysiological effect on man in the above methods, however, is limited by control of alpha activity which does not allow to effectively correct functional condition of the body.

Also known is the method of body functional condition correction with optimization of parameters of external effect on the body which includes recording of physiological parameter biopotentials, transformation and processing of the obtained information with calculation of a biosignal characteristic parameter which is transformed into a control signal, and external effect signals are formed on the basis of the data obtained (see USSR Authorship Certificate No. 1745204, class A 61 B 5\04, 1992).

In this case the external effect, e.g. background sound, is selected from various prerecorded phonograms that differ in volume, rhythm, and tone, using biological feedback to optimize deviation of current characteristic value of the selected biosignal registered during correction of patient's functional condition from the estimated one determined in the preparatory mode. These prerecorded phonograms, however, are of random nature and may not fully correspond to individual features of the body, which reduces effectiveness of man's physiological condition psychophysiological correction by external effect of physical factors, e.g. sound.

Document EP-A-0 301 790 discloses a method and apparatus according to the preamble of claims 1 and 3, respectively.

Disclosure of the Invention

The object of the invention is that a method is provided which can register a sound which is capable to influence the body by means of an external physical factor - a sound in the form of a musical tune that adequately reflects man's psychophysiological condition. A further object is the provision a respective device for carrying out this method.

The solution of the problem is provided by the method and the device of the present invention as defined in claims 1 and 3, respectively.

A positive outcome of the method which can be carried out with the claimed device is primarily that sound reproduction of physiological activity biosignals is based on the analogy of oscillatory nature of recorded biosignal variation (electroencephalogram - EEG, electrocardiogram - ECG, electrogastrogram - EGG, electromyogram, electroretinogram, pulse wave oscillogram, etc.) and sound oscillatory nature, while suggested criterial dependence between characteristic generalized parameter of frequency spectrum of transformed biosignal and parameters of generated musical sound (tone, volume, and duration) most adequately reflects individual features of man's functional condition and allows to form sequence of sounds in the form of personality music which, if recorded in magnetic medium while the patient is in healthy condition, allows to effectively correct depressive conditions, sleep disturbance, anxiety and other psychophysiological disorders by music therapy.

Brief Description of Drawings

Fig. 1 shows the set of frequency spectra with discrete current alternation of time intervals.

Fig. 2 shows range of musical sound parameters.

The Best Embodiment of the Invention

The method suggested is implemented as follows.

Preliminary, during a satisfactory period of patient's healthy condition, physiological parameter biopotentials, e.g. bioelectric activity of brain, heart muscles, stomach, skeletal muscles, eye retina, pulse waves, etc., are recorded using well-known advanced instrumentation.

Electroencephalogram, EEG, is the most universal and adequately reflects individual functional condition; an example of EEG transformation into the "brain music" is given below.

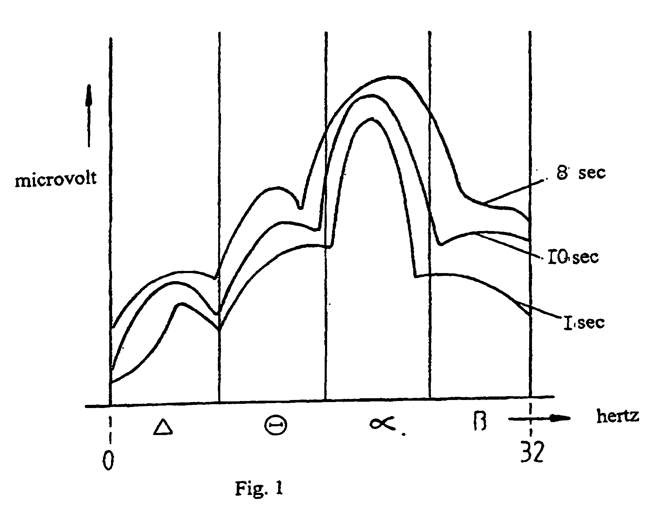

EEG registered e.g. within 10 seconds, is divided into equal time intervals of, e.g. 1 second duration; using harmonic analysis, a Fourier expansion, each interval is transformed into frequency spectrum (see Fig. 1), 4 common frequent ranges ($\Delta$, $\Theta$, $\alpha$, $\beta$) are isolated pursuant to the international standard:

$$\Delta = 0.1\text{-}3.9 \text{ Hz},$$

$$\Theta = 4.0\text{-}4.7 \text{Hz}$$

$$\alpha = 8.0\text{-}12.9 \text{ Hz},$$

$$\beta = 13.0 \text{ - } 32.0 \text{ Hz},$$

and a dimensionless generalized characteristic parameter is determined for each spectral interval with respect to power density of the spectra of $\alpha$ and $\beta$ intervals, namely:

$$K_1 = \frac{P_1\Theta}{P_1\beta} = 30.0 = \frac{15.0}{0.5} \text{ (for 1st second)}$$

$$K_2 = \frac{P_2\Theta}{P_2\beta} = 20.0 = \frac{42.0}{2.1} \text{ (for 2nd second)}$$

$$K_3 = \frac{P_3\Theta}{P_3\beta} = 12.0 = \frac{54.0}{4.5} \text{ (for 3rd second)}$$

$$K_4 = \frac{P_4\Theta}{P_4\beta} = 20.0 = \frac{76.0}{3.8} \text{ (for 4th second)}$$

$$K_5 = \frac{P_5\Theta}{P_5\beta} = 11.0 = \frac{81.4}{7.4} \text{ (for 5th second)}$$

$$K_6 = \frac{P_6\Theta}{P_6\beta} = 10.0 = \frac{105.0}{10.5} \text{ (for 6th second)}$$

$$K_7 = \frac{P_7\Theta}{P_7\beta} = 93 = \frac{78.4}{0.8} \text{ (for 7th second)}$$

$$K_8 = \frac{P_8\Theta}{P_8\beta} = 5.5 = \frac{101.8}{18.5} \text{ (for 8th second)}$$

$$K_9 = \frac{P_9\Theta}{P_9\beta} = 8.5 = \frac{51.0}{6.0} \text{ (for 9th second)}$$

$$K_{10} = \frac{P_{10}\Theta}{P_{10}\beta} = 6.0 = \frac{135.5}{12.5} \text{ (for 10th second),}$$

where

K - dimensionless generalized parameter;
$P\Theta$, $P\beta$ - power density of the spectra of characteristic frequency bands (sq.$\mu$V/sec).

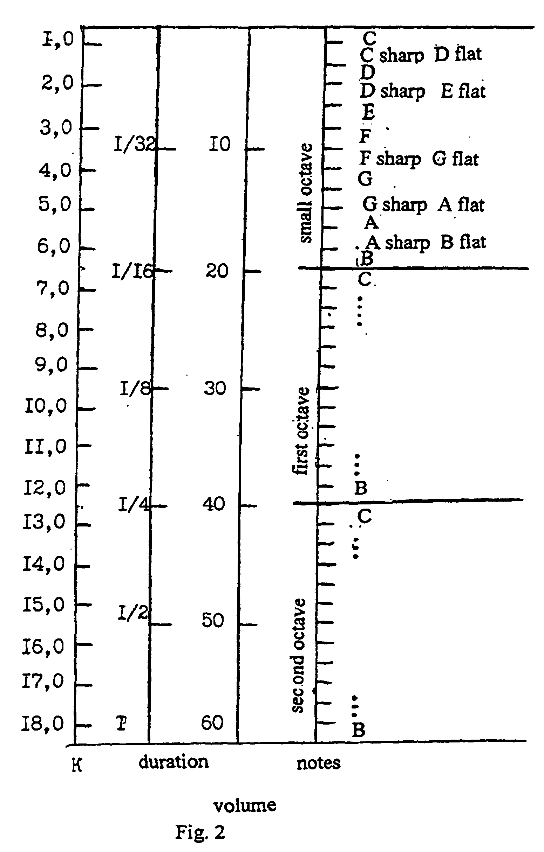

On the basis of calculation results, determined is a numerical interval between minimum ($K_1 = 0.5$) and maximum ($K_8 = 18.5$) values of generalized characteristic parameter, where drawn is a proportional range of musical sound parameters including 36 notes of three octaves (small, first and second) for piano, 8 volume gradations, and 8 duration segments (see Fig. 2), which reflects criterial relation among them. Numerical value of generalized dimensionless parameter of each spectral interval is used to determine appropriate parameters of musical sound which in the sequence appropriate to originally recorded discrete current alternation of time intervals are transformed (sonified) by means of a sound card (synthesizer) into "brain music" which is recorded on magnetic medium.

The external sound effect - music therapy according by using the claimed device was successfully used for treatment of sleep disturbance in over 200 patients as follows.

Patients with sleep disturbance usually have decreased sleep duration, prolonged dormition, increased vigil time inside sleep and increased number of awakenings, increased duration of sleep surface stages (1 st and 2nd stages) and decreased delta-sleep and REM duration.

To correct sleep, sleep polygram including electroencephalogram is recorded at satisfactory moment; 2nd stage, delta-sleep and REM sections according to international criteria are selected in BEG, transformed the recorded information pursuant to the method into "brain music", an obtained composition is recorded in magnetic medium. In the process of correction, each patient listens to his/her "brain music" every night in bed for 15 days, which significantly improves subjective characteristic of sleep and objective sleep structure.

Industrial Applicability

Hardware implementation of the method claimed may involve a personal computer with use of advanced electronic instrumentation - electroencephalograph, tape recorder, etc.

**Claims**

1. A method for registering a musical sound useful for influencing the body of an individual comprising registration of physiological parameter biopotentials during a healthy condition of the individual, transformation and processing of the obtained data with calculation of a biosignal characteristic generalized parameter, which on the basis of detected criterial correspondence is transformed into a control signal and forms an external sound effect, comprising the steps of:

- registering of physiological biopotentials which are represented by a respective graphic information;

- dividing the registered graphic information into time intervals of identical duration which are transformed using Fourier harmonic analysis into a frequency spectrum;

- determining for each time interval of the frequency spectrum generalized dimensionless parameters;

- forming a range of musical sound parameters proportional to the numeric interval between minimum and maximum values of the generalized dimensionless parameter of the interval of the frequency spectrum;

- determining and transforming the musical sound parameters of the numerical value of the generalized dimensionless parameter values of each interval of the frequency spectrum by using a synthesizer to sound signals which are formed in sequence appropriate to originally recorded discrete current alternation of time intervals; and

- varying the tone, volume and duration in relation to variation of the discrete current value of characteristic generalized parameters of the frequency spectrum,

**characterized in that** the generalized dimensionless parameters are determined by the ratio of power densities of the spectrum of two characteristic frequency bands selected in each spectral interval.

2. The method for registering a musical sound according to claim 1 **characterized in that** the physiological parameter biopotentials are electroencephalograms, electrocardiograms, electrogastrograms, electromyograms, electroretinograms or pulse wave oscillograms reflecting the bioelectric activity of brain, heart muscles, stomach, skeletal muscles, eye retina or pulse waves.

3. A device for registering a musical sound useful for influencing the body of an individual according to the method of claim 1 comprising

- a means for recording biopotentials ot the physiological parameters represented by a respective graphic information;
- a means for dividing the graphic information in-

to equal time intervals;

- a means for the transformation of the time intervals into a frequency spectrum by Fourier harmonic analysis;

- a means for determining for each time interval of the frequency spectrum generalized dimensionless parameters;

- a means for forming a range of musical sound parameters proportional to the numeric interval between minimum and maximum values of the generalized dimensionless parameter of the interval of the frequency spectrum;

- a synthesizer for determining and transforming of the musical sound parameters for each generalized dimensionless characteristic parameter of every time interval to sound signals which are formed in sequence appropriate to originally recorded discrete current alternation of time intervals;

- a means for varying the tone, the volume and the duration of the sound signals in relation to variation of the discrete current value of characteristic generalized parameters of the frequency spectrum; and,

- a recording means for recording the generated musical sound,

**characterized in that** the generalized dimensionless parameters are determined by the ratio of power densities of the spectrum of two characteristic frequency bands selected in each spectral interval.

**Patentansprüche**

1. Verfahren zur Aufzeichnung von einem Musikton zur Beeinflussung des Körpers eines Individuums umfassend die Aufzeichnung von physiologischen Parameter-Biopotentialen bei einem gesunden Zustand des Individuums, die Verwandlung und Verarbeitung der erhaltenen Daten mit Berechnung eines Biosignal-charakteristischen verallgemeinerten Parameters, der basierend auf der erfassten Kriterienübereinstimmung in ein Kontrollsignal umgewandelt ist und einen externen Toneffekt bildet, wobei das Verfahren folgende Schritte umfasst:

- Aufnahme von physiologischen Biopotentialen, die durch eine jeweilige graphische Angabe dargestellt sind;

- Aufteilung der erfassten graphischen Angabe in Zeitabständen gleicher Dauer, die in ein Frequenzspektrum unter Verwendung der Fourier harmonischen Analyse umgewandelt sind;

- Ermittlung der dimensionslosen verallgemeinerten Parameter zu jedem Zeitabstand des Frequenzspektrums;

- Bildung eines Bereichs von Musiktonparametern im Verhältnis zum numerischen Abstand zwischen minimalen und maximalen Werten der dimensionslosen verallgemeinerten Parameter des Abstands des Frequenzspektrums;

- Ermittlung und Umwandlung von den Musikton-Parametern des numerischen Wertes der dimensionslosen verallgemeinerten Parameterwerte von jedem Abstand des Frequenzspektrums unter Verwendung von einem Synthesizer in Tonsignale, die in Sequenzen gebildet sind, die für die ursprünglich aufgezeichnete diskrete aktuelle Wechselfolge von Zeitabständen geeignet sind, und

- Veränderung des Tons, der Lautstärke und der Dauer relativ zu der Veränderung des diskreten aktuellen Werts der verallgemeinerten charakteristischen Parameter des Frequenzspektrums,

  **dadurch gekennzeichnet, dass** die dimensionslosen verallgemeinerten Parameter durch das Verhältnis von Leistungsdichten des Spektrums von zwei in jedem spektralen Abstand ausgewählten charakteristischen Frequenzbändern ermittelt werden.

2. Verfahren zur Aufzeichnung von einem Musikton nach Anspruch 1, **dadurch gekennzeichnet, dass** die physiologischen Parameter-Biopotentialen Elektroenzephalogramme, Elektrokardiogramme, Elektrogastrogramme, Elektromyogramme, Elektroretinogramme oder Pulswellen-Oszillogramme, die die bioelektrische Aktivität, des Gehirns, der Herzmuskeln, des Magens, der Skelettmuskeln, der Netzhaut oder Impulswellen veranschaulichen.

3. Vorrichtung zur Aufzeichnung von einem Musikton zur Beeinflussung des Körpers eine Individuums nach Anspruch 1, umfassend

- Mittel zur Aufzeichnung von Biopotentialen der physiologischen Parameter, die durch eine jeweilige graphische Angabe dargestellt sind,

- Mittel zur Aufteilung der graphischen Angabe in gleichen Zeitabständen;

- Mitteln zur Umwandlung der Zeitabständen in ein Frequenzspektrum durch die harmonische Fourier-Analyse;

- Mittel zur Ermittlung der dimensionslosen verallgemeinerten Parameter zu jedem Zeitabstand des Frequenzspektrums;

- Mittel zur Bildung eines Bereichs von Musiktonparametern im Verhältnis zum numerischen Abstand zwischen minimalen und maximalen Werten der dimensionslosen verallgemeinerten Parameter des Abstands des Frequenzspektrums;

- einen Synthesizer zur Ermittlung und Umwandlung von den Musikton-Parametern für jeden dimensionslosen verallgemeinerten Parameter von jedem Zeitabstand in Tonsignale, die in Sequenzen gebildet sind, die für die ursprünglich aufgezeichnete diskrete aktuelle Wechselfolge von Zeitabständen geeignet sind;

- Mittel zur Veränderung des Tons, der Lautstärke und der Dauer der Tonsignale relativ zu der Veränderung des diskreten aktuellen Werts der verallgemeinerten charakteristischen Parameter des Frequenzspektrums, und

- Aufzeichnungsmittel zur Aufzeichnung des hergestellten Musiktons,

  **dadurch gekennzeichnet, dass** die dimensionslosen verallgemeinerten Parameter durch das Verhältnis von Leistungsdichten des Spektrums von zwei in jedem spektralen Abstand ausgewählten charakteristischen Frequenzbändern ermittelt werden.

## Revendications

1. Procédé pour l'enregistrement d'un son musical utilisé pour influencer le corps d'un individu comprenant l'enregistrement de biopotentiels de paramètres physiologiques dans une condition de bonne santé de l'individu, la transformation et le traitement des données obtenues avec calcul d'un paramètre généralisé caractéristique de biosignal qui est transformé sur la base de la concordance de critères en un signal de contrôle et forme un effet sonore externe, comprenant les étapes suivantes :

- enregistrement de biopotentiels physiologiques qui sont représentés par une information graphique respective;

- division de l'information graphique enregistrée en intervalles de temps de durée identique qui sont transformés en utilisant l'analyse harmonique de Fourier en un spectre de fréquences;

- Détermination de paramètres généralisés sans dimension pour chaque intervalle de temps du spectre de fréquences;

- Formation d'une plage de paramètres de son musical proportionnellement à l'intervalle numérique entre les valeurs maximales et minimales des paramètres généralisés sans dimension de l'intervalle du spectre de fréquence;

- Détermination et transformation des paramètres de son musical de la valeur numérique des valeurs des paramètres généralisés sans dimension de chaque intervalle du spectre de fréquences en utilisant un synthétiseur, en signaux sonores qui sont formés en séquence appropriée à l'alternance actuelle discrète enregistrée à l'origine d'intervalles de temps; et

- variation du son, du volume et de la durée en relation avec la variation de la valeur courante discrète des paramètres généralisés sans dimension du spectre de fréquences,

  **caractérisé en ce que** les paramètres généralisés sans dimension sont déterminés par le rapport des densités de puissance du spectre de deux bandes de fréquences caractéristiques sélectionnées dans chaque intervalle du spectre.

2. Procédé pour l'enregistrement d'un son musical selon la revendication 1, **caractérisé en ce que** les paramètres physiologiques sont des électroencéphalogrammes, des électrocardiogrammes, des électrogastrogrammes, des électromyogrammes, des électrorétinogrammes ou des oscillogrammes d'ondes de pression reflétant l'activité bioélectrique du cerveau, des muscles cardiaques, de l'estomac, des muscles squelettiques, de la rétine oculaire ou des ondes de pression.

3. Dispositif pour l'enregistrement d'un son musical utilisé pour influencer le corps d'un individu selon le procédé de la revendication 1, comprenant

- un moyen pour l'enregistrement de biopotentiels physiologiques qui sont représentés par une information graphique respective;

- un moyen pour la division de l'information graphique enregistrée en intervalles de temps de durée identique

- un moyen pour la transformation des intervalles de temps en un spectre de fréquences en utilisant l'analyse harmonique de Fourier;

- un moyen pour la détermination de paramètres généralisés sans dimension pour chaque intervalle de temps du spectre de fréquences;

- un moyen de formation d'une plage de paramètres de son musical proportionnellement à l'intervalle numérique entre les valeurs maximales et minimales des paramètres généralisés sans dimension de l'intervalle du spectre de fréquence;

- un synthétiseur pour la détermination et la transformation des paramètres de son musical pour chaque paramètre caractéristique généralisé sans dimension de chaque intervalle de temps en signaux sonores qui sont formés en séquence appropriée à l'alternance actuelle discrète enregistrée à l'origine d'intervalles de temps; et

- un moyen de variation du son, du volume et de la durée en relation avec la variation de la valeur courante discrète des paramètres généralisés sans dimension du spectre de fréquences,

  **caractérisé en ce que** les paramètres généralisés sans dimension sont déterminés par le rapport des densités de puissance du spectre de deux bandes de fréquences caractéristiques sélectionnées dans chaque intervalle du spectre.

Fig. 1

7

Fig. 2